# EUROPEAN PATENT APPLICATION

(11) **EP 1 716 812 A1**
(43) Date of publication of application: **02.11.2006**
(21) Application number: 06008466.2
(22) Date of filing: 25.04.2006
(51) Int. Cl.: A61B 17/068, B25C 5/11

(54) **Medical stapler**

(30) Priority: 28.04.2005 JP 2005132569
(71) Applicant: Mani, Inc., Shioya-gun, Tochigi-ken (JP)
(72) Inventor: Matsutani, Kanji, Shioya-gun Tochigi-ken (JP); Kamei, Toshiharu, Shioya-gun Tochigi-ken (JP); Fukuda, Masatoshi, Shioya-gun Tochigi-ken (JP)
(74) Representative: Patentanwälte Westphal, Mussgnug & Partner

(57) **Abstract**

The present invention relates to a medical stapler for suturing body tissue by forming a medical staple (3) supported by an anvil (5) by bringing a ram (6) close to the anvil, comprising a housing (1), a lever (2), an anvil and a ram, and the ram is composed of metal material and the drive portion (2a) formed on part of the lever and the ram guide portion (9) provided in the housing are composed of synthetic resin and a gap between a leg formed on the ram and the anvil opposing the leg is set to a dimension allowing the medical staple to be formed into an excellent shape with a light load.

## Description

### BACKGROUND OF THE INVENTION

### 1. Field of the Invention

The present invention relates to a medical stapler used for suturing body tissues and more particularly to a medical stapler constructed to reduce a force necessary for forming a staple.

### 2. Description of the Related Art

In surgical operation, incised body tissues are sutured using the medical stapler. In this medical stapler, a medical staple formed into a U shape using metallic linear material as its raw material is supported on an anvil and a ram is brought close to the anvil so as to make legs formed on the ram into contact with both end portions of the crown of the medical staple and further, by moving the ram, the medical staple is formed into a square shape by cooperative actions of the ram and anvil so as to suture the body tissues on this forming process.

The medical stapler is so constructed to contain a predetermined quantity of the medical staples in an accommodating portion provided in its housing and be capable of urging the medical staple in a direction of the anvil and ram. It has a lever which is rotated by an operation of a medical doctor and the ram or the ram and anvil are moved in the direction of a body tissue to be sutured by a drive portion formed in part of the lever (for example, see Japanese PatentApplicationLaid-Open No.2000-217829).

Some medical stapler is so constructed that the medical staple is supplied from the accommodating portion to the anvil formed unmovable to the housing and thereby supporting the staple. The medical stapler having such a structure can form the medical staple by bringing the ram to the anvil supporting the medical staple.

In other medical stapler, a plate-like member provided with an anvil is accommodated in a housing such that it can move to a suture site, while a ram composed of a plate-like material is accommodated such that it can move to the anvil and the suture site. In the medical stapler having such a structure, a medical staple is received with the anvil facing the accommodating portion and can be nipped between the ram and the anvil by moving only the ram under this condition. As a consequence, the medical staple can be formed by moving the ram to the anvil at the same time when the anvil and the ram are brought near to a suturing object area.

Various resistances occur when the body tissue is sutured by operating the medical stapler. Such various resistances include friction resistance generated between a drive portion formed on part of the lever and the ram, friction resistance generated between the ram and the guide portion for guiding the motion of the ram when the rammoves, pierce resistance generated when the medical staple pierces the body tissue, resistance of material when the medical staple is formed, resistance due to contact resistance between the ram and the medical staple and the like. Although sites which make sliding contact with each other are preferred to be lubricated, it is difficult to lubricate the medical stapler sufficiently. As a consequence, this can cause a medical doctor to be fatigued with an increase of the quantity of suturing with such a resistance received.

Particularly, the anvil is formed on a metallic plate-like member disposedmovablyto the housing and the ram is also composed of a metallic plate-like member of the same material as the anvil constructed to be movable to the housing and the plate-like member. As a consequence, when the ram moves relative to the anvil to form the medical staple, the metal members of the same material come to make sliding contact with each other in non-lubricating condition whereby producing a large friction resistance.

When the lever is operated to suture the body tissue, a drive action of applying a force to part of the lever with the ram making contact with the part of the lever is generated. If the lever is composed of synthetic resin, distortion can be generated in the face of a drive portion by the force applied to the ram and to prevent this distortion, conventionally, the portion making contact with the ram in the drive portion is reinforced by forming a metal plate of the same material as the ram integrally. However, with this structure, the metals of the same material comes to make sliding contact with each other thereby generating a large friction resistance.

Even if the lever of synthetic resin presses the ramthrough a member formed of synthetic resin of the same material, it comes that the synthetic resins of the same material make sliding contact with each other thereby relatively increasing friction resistance. Although when the synthetic resins are brought into sliding contact with each other, the friction resistance can be reduced by using synthetic resin material having self-lubricating characteristic, such synthetic resin material is expensive so that the cost of the medical stapler is increased, which is a problem to be solved.

Because the medical staple which sutures the body tissue more hardly leaves when it is bent at right angle at its formation portion than when it is bent curvedly, weight has been placed on forming the staple at right angle for the medical stapler but the magnitude of force necessary for the formation has not attracted much attention.

### SUMMARY OF THE INVENTION

An object of the present invention is to provide a medical stapler which enables a force necessary for forming a medical staple to be reduced.

To achieve the above-described object, the present invention provides a medical stapler for suturing body tissue by forming a medical staple supported by an anvil by bringing a ram close to the anvil, including: a housing provided with a ram guide portion for guiding the ram in the direction of the anvil and equipped with a lever such that it is rotatable; a lever in which a driveportion formed in part thereof makes contact with the ram so as to bring the ram close to the anvil accompanied by a rotation of the lever; an anvil that is fixed to the housing and continuous to the front end of an accommodating portion for accommodating a plurality of medical staples so as to support a medical staple supplied from the accommodating portion; and a ram that is constructed to be capable of sliding with respect to the housing and reciprocating with respect to the anvil and has a contact portion making contact with the drive portion formed in part of the lever and a pair of legs formed on an end portion for forming the medical staple in the anvil, wherein the ram is composed of metal material and the drive portion formed on part of the lever and the ram guide portion provided in the housing are composed of synthetic resin and a gap between a leg formed on the ram and the anvil opposing the leg is set to a dimension allowing the medical staple to be formed into an excellent shape with a light load.

Because in the medical stapler (hereinafter, simply referred to as stapler) of the present invention, the anvil is fixed to the housing such that it is continuous to the front end of the accommodating portion for accommodating the medical staple (hereinafter, simply referred to as staple) and the ram is composed of metal, no sliding friction occurs between the ram and the anvil. Further because the drive portion formed on part of the lever which is a site the ram makes contact with when the ram moves in the direction of the anvil and the guide portion of the ram formed in the housing are composed of synthetic resin, the sliding friction resistance decreases so that no large force is necessary as the hardness of materials (ram composed of metal and drive portion and guide portion which make contact with the ram, composed of synthetic resin) at a site which the ram makes contact with. Thus, the force necessary for suturing the body tissue can be reduced.

When the ram and the drive portion formed on part of the lever and the ram and the guide portion make sliding contact with each other respectively, the contact face on the synthetic resin side follows up the contact face of metal because of a difference in hardness, so that a smooth sliding contact is achieved and the friction resistance can be reduced.

By setting the gap between the leg formed on the ram and the end portion of the anvil to a dimension corresponding to the size of the staple, the staple can be bent at right angle with a minimum force. Thus, the fatigue of a medical doctor can be reduced.

### BRIEF DESCRIPTION OF THE DRAWINGS

FIG. 1 shows three type of views of a stapler in a condition in which its lever is not operated;
FIG. 2 is a side view of the stapler when a staple is formed by operating the lever; and
FIGS. 3A and 3B are diagrams for explaining the order of processes supported by an anvil of forming the staple.

### DESCRIPTION OF THE PREFERRED EMBODIMENTS

Hereinafter, the preferred embodiment of the stapler of the present invention will be described. The stapler of the present invention can reduce a force necessary for suturing the body tissue by forming a U-shaped staple supported by the anvil into a square shape and form the staple into a reasonable shape.

Thus, the ram is formed of a metallic plate member and a contact portion between a drive portion and the ram, provided on part of a lever and a guide portion for guiding a moving direction of the ram when the ram moves to the anvil are formed of synthetic resin respectively so as to reduce resistance due to contact friction. Because the ram is formed of the metallic plate, and the lever drive portion which makes sliding contact with the ram and the guide portion provided in the housing are formed of synthetic resin, the friction resistance of this embodiment is smaller than friction resistance generated between one and anothermetals or between one and another synthetic resins of the same material thereby making it possible to reduce the necessary force.

By setting a gap between a leg provided on the ram and an end portion of the anvil, correspondingly to the thickness of a staple, the formation shape of the staple can be secured in an excellent one and resistance upon formation can be reduced. Because the gap between a leg forming face provided on the ram and the end portion of the anvil is set corresponding to the thickness of the staple, the staple can be formed into an excellent shape and at the same time, resistance upon formation can be reduced.

The metal material for constituting the ram is not restricted to any particular one but any metal material may be used as long as it has enough strength resisting to force acting when the staple is formed. However, generation of no rust is preferable from viewpoints of a medical device. Thesematerials include stainless steel, particularly austenitic stainless steel and it is preferable to use austenitic stainless steel plate member whose strength is intensified by cold plastic forming.

Although the material of synthetic resin which constitutes the drive portion formed on a lever making a sliding contact with the ram and the guide portion provided in the housing is not restricted to any particular one but it is preferred to have self-lubricating characteristic. However, balance with cost is an important matter and even if the self-lubricating characteristic is not possessed, resistance due to sliding friction can be set smaller than sliding friction generated between one andanothermetal or between one and another synthetic resin of the same material.

The gap between the leg provided on the ram and the end portion of the anvil is set corresponding to the thickness of the staple. When staples accommodated in an accommodating portion are consumed completely, no new staples are replenished to this stapler even during suturing operation and in this case, a new stapler is used. Thus, the size of the gap between the leg of each stapler and the end portion of the anvil is constant.

### [A]

Herein, "The gap between the leg of the ram and the end portion of the anvil in the stapler" refers to a distance between a substantially outer face of the formation face on the leg of the ram which the staple makes contact with and the end portion of the anvil. That is, the formation face includes a flat plane, a recessed plane, a plane including a V-shaped groove and the like.

### [Embodiment 1]

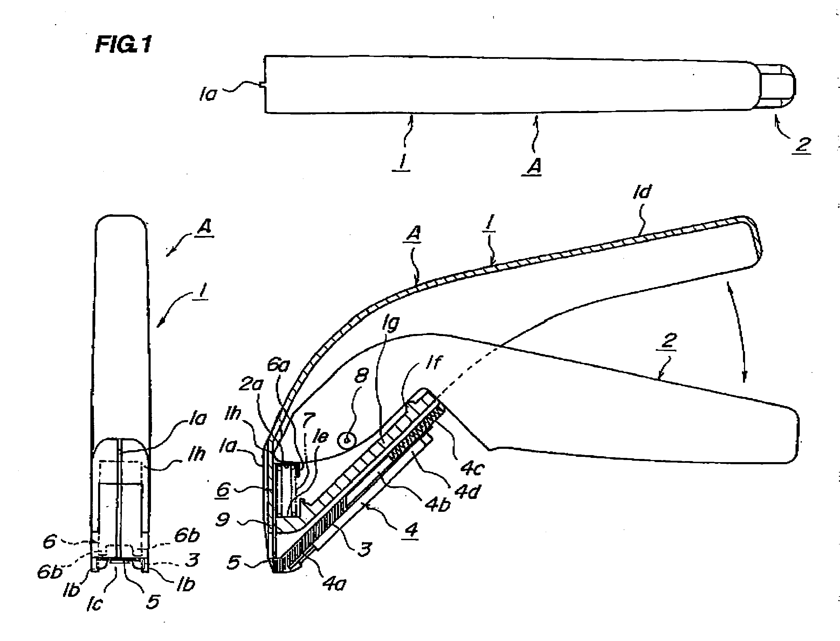

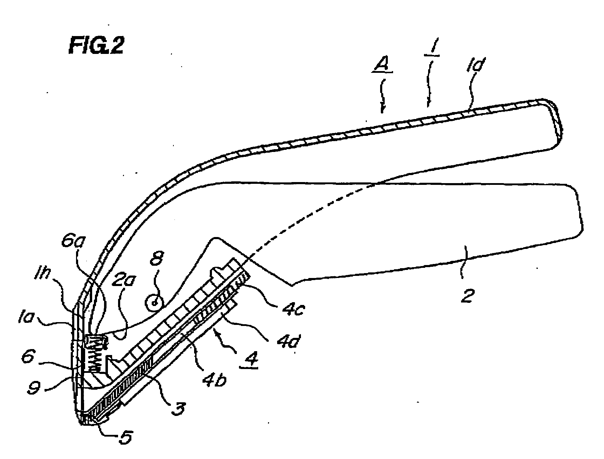

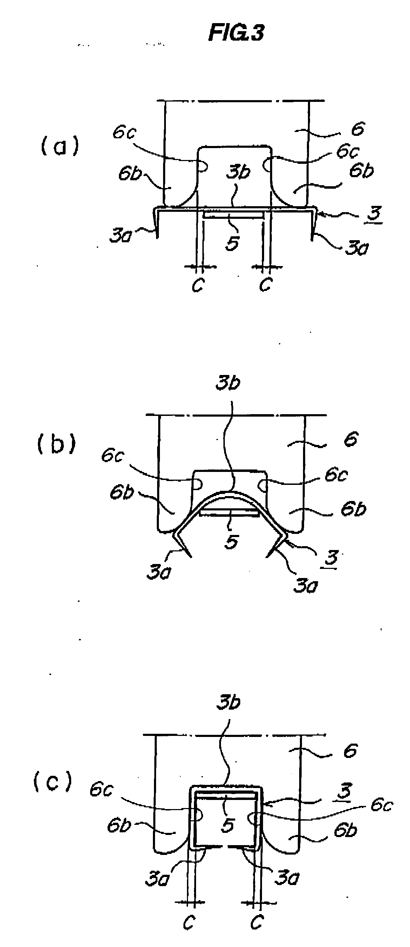

Hereinafter, the structure of the stapler according to the first embodiment will be described with reference to the accompanying drawings. FIG. 1 shows three types of views of a stapler in a condition in which the lever is not operated. FIG. 2 is a side view of the stapler when the staple is formed by operating the lever. FIG. 3 is a diagram for explaining the order of processes for forming the staple supported by the anvil.

The stapler A shown in the Figure has a housing 1, a lever 2 mounted rotatably to the housing 1 and equipped with a drive portion 2a at a part thereof (one end thereof in the present embodiment), an accommodating portion 4 constituted integrally with the housing 1 for accommodating a plurality of staples 3, an anvil 5 disposed continuous to the accommodating portion 4 and constructed immovably to the housing 1, a ram 6 disposed movably in the direction of the anvil 5 and supported by the anvil 5 for forming a staple 3 and an urging member 7 for urging the ram 6 in the direction of the drive portion 2a of the lever 2.

The housing 1 is formed of synthetic resin (ABS resin in this embodiment) and molded into a shape considering ease of gripping and operability together with the lever 2. A protrusion 1a indicating the center is formed on the front face of the housing 1 and this protrusion 1a enables a reference upon suturing the incised site to be recognized. A contact piece 1b which makes contact with both end portions of the staple 3 supported by the anvil 5 for preventing the staple 3 from leaving the stapler A is formed at the bottom on the front face of the housing 1. Further, an opening 1c which allows the staple 3 formed in the center of the contact piece 1b to leave the anvil 5 (from the stapler A) is formed.

A shaft 8 is disposed at a predetermined position of the housing 1 and the lever 2 is mounted rotatably on the shaft 8. The section of a portion corresponding to the rotation range of the lever 2 in the housing 1 is formed in a U shape, so that this shape can receive the lever 2 when the lever 2 is operated and rotated. A flange 1g having a seat 1e of the urging member 7 and a stopper 1f which specifies the rotation limit of the lever 2 is formed at a position corresponding to the accommodating portion 4 of the housing 1.

A guide portion 9 for guiding the motion of the ram 6 is formed between the inner side of a front face wall 1h of the housing 1 and the flange 1g formed in the housing 1. This guide portion 9 is formed of a groove meeting the thickness of the ram 6 and by fitting both end portions in the width direction of the ram 6, the motion of the ram 6 can be guided in directions of approaching the anvil 5 or leaving it. Particularly because the ram 6 is formed of metal while the guide portion 9 is formed of synthetic resin (ABS resin), the friction resistance when the ram 6 moves so that it slides along the guide portion 9 is smaller enough than friction resistance when one and another metals or one or another synthetic resins of the same material make sliding contact with each other in non-lubricating condition.

The lever 2 is formed of synthetic resin (ABS resin in this embodiment) as well as the drive portion 2a which is driven in contact with the ram 6 and the angle between the housing 1 and the lever 2 and the like in a free condition is set considering the ease of grip property and operability. Further, because a large force is applied to the lever 2 when the body tissue is sutured with the staple 3, a plurality of ribs, flanges and the like are formed around the shaft 8 as well as the end portions including the drive portion 2a in order to be capable of exerting a high stiffness. Provision of the ribs and flanges can prevent the lever from being deformed even when a large force is applied to the lever, particularly can prevent generation of a bending which can occur in the drive portion 2a if such a measure is not taken.

The staple 3 is formed in a U shape of linear material of stainless steel and its both end portions are formed into a sharp pointed ends 3a to reduce resistance when the body tissue is pierced by the both end portions and a crown 3b is formed in the center. The crown 3b is a portion which is bent when the body tissue is sutured.

The staples 3 are accommodated in the accommodating portion 4 such that they are arranged in a predetermined quantity. This accommodating portion 4 is composed of a holding portion 4a whose front end is continuous to the anvil and in which a plurality of the staples 3 are arranged in line, a contact member 4b which makes firm contact with the staples 3 held in the holding portion 4a, an urging member 4c for urging the staples 3 in the direction of the contact piece 1b of the housing through the contact member 4b and a casing 4d which accommodates the holding portion 4a, the contact member 4b and the urging member 4c.

The casing 4d constituting the accommodating portion 4 is formed integrally with the housing 1 or formed as an isolated body and fixed to the housing 1 by fitting thereto. According to this embodiment, the casing 4d is formed of transparent ABS resin so that the quantity of left staples 3 accommodated in the accommodating portion can be recognized from outside.

The holding portion 4a has a function of holding a preset quantity of the staples 3 and a function of guiding a staple 3 urged by the urging member 4c when a staple located at the head is formed and leaves the anvil 5. Therefore, the holding portion 4a may be constructed in any shape as long as it is constructed to be able to exert those functions and the shape and material of the holding portion 4a are not restricted to any particular ones.

According to this embodiment, the holding portion 4a and the anvil 5 are constructed integrally so that they are continuous in order to enable supply of the staple 3 from the holding portion 4a to the anvil 5 to be carried out smoothly. Because a high stiffness and strength are required for the anvil 5 to exert the function of supporting the staple 3, the holding portion 4a and the anvil 5 are constructed by forming a single stainless steel into a Ω shape. The holding portion 4a and the anvil 5 constructed in this way are fixed using an undercut portion formed in the casing 4d constituting the accommodating portion 4.

The contact portion 4b is formed into the same shape as the staple 3 and disposed in the holding portion 4a so that it keeps contact with the staple 3 located at a rearmost position. The urging member 4c, for example, a compression spring is connected to the contact member 4b, so that the staple 3 is urged in the direction of the anvil 5 by the contact member 4b.

The anvil 5 is formed to be continuous to the front end of the holding portion 4a and disposed in the opening portion 1c provided in the front face of the housing 1. Because the holding portion 4a is fixed to the casing 4d and casing 4d is constructed integrally with the housing 1, the anvil 5 is fixed to the housing 1.

The width dimension of the anvil 5 corresponds to the lateral dimension of a square defined when the staple 3 is formed. Particularly, because the anvil 5 is formed in the shape of a cantilever from the holding portion 4a constituting the accommodating portion 4, it is formed with a thickness capable of resisting sufficiently a force acting when the staple 3 is formed.

The ram 6 is driven by the lever 2 and moved in the direction of the anvil 5 and in this moving process, the staple 3 supported by the anvil 5 is formed. The top end portion of the ram 6 is bent substantially at right angle and the drive portion 2a of the lever 2 makes a contact with the top face thereof while a contact piece 6a in which a seat for the urging member 7 is formed on the bottom face thereof. A pair of legs 6b are formed on the bottom end portion and on both sides in the width direction of the ram 6.

When the lever 2 is rotated counterclockwise in FIG. 1 by applying a force to the lever 2, the ram 6 is driven and moved to the anvil 5 (downward) in accordance with this rotation. When the force applied to the lever 2 is released, the ram 6 is urged by the urging member 7 and then moved in the direction of leaving the anvil 5 (upward). Accompanied by moving upward of the ram 6, the lever 2 is urged and rotated clockwise.

Because the contact piece 6a of the ram 6 keeps contact with the drive portion 2a of the lever 2, the drive portion 2a makes sliding contact with the contact piece 6a accompanied by a rotation of the lever 2. Because the drive portion 2a is formed of synthetic resin although the contact piece 6a is formed of metal, the friction resistance by the sliding contact is sufficiently smaller than the friction resistance when one and another metals or one and another synthetic resins of the same material make sliding contact in non-lubricating condition.

Because the ram 6 is formed of metal and the drive portion 2a and the guide portion 9 are formed of synthetic resin, the friction resistance is reduced. As a result of considering about that a force necessary for forming the staple is reduced by comparing with other staplers, a following result is obtained.

As regards the stapler A of this embodiment and staplers B, C (not shown) manufactured by other companies, three points, that is, (I) a magnification (power point magnification) from a rotation shaft (fulcrum point) up to a grip portion (power point) when a distance from the rotation shaft (fulcrum point) of the lever to the drive portion (action point) which drives the ram is assumed to be 1, (II) materials of the drive portion and ram guide portion and (III) maximum load (formation load) when the respective staplers A-C form each staple, were considered by comparison. In the respective staplers A-C, each ram is composed of metal and the size of the staple is 0.58 mm.

As regards the power point magnification of (I), the stapler A of this embodiment indicated 4.18 times, the stapler B of other company indicated 3.90 times and the-stapler C of other company indicated 5.94 times.

As regards the material of (II), in case of the stapler A of this embodiment, its drive portion and guide portion are composed of synthetic resin. In the stapler B of other company, its drive portion is composed of synthetic resin and constructed to drive the ram through other component composed of synthetic resin while the guide portion is composed of synthetic resin together with a metallic anvil. In the stapler C of other company, its drive portion and guide portion are composed of synthetic resin. Because the ram is fixed to a fixing component of synthetic resin and that fixing component slides on the guide portion, it comes that the synthetic resins make sliding contact with each other.

As regards the formation load of (III), the stapler A of this embodiment indicated 1.97 kgf, the stapler B of other company indicated 2. 46 kgf and the stapler C of other company indicated 2.60 kgf.

As described above, although the power point magnification of the stapler B of other company was relatively near the stapler A of this embodiment, its formation load was about 1.25 times larger than the stapler A. Further although it was estimated that the formation load of the stapler C of other company was smaller than the stapler A because the power point magnification of the stapler C is much larger than the stapler A of this embodiment, it was larger than the stapler A actually. Because the sizes of the staples are equal for the staplers A-C, if it is assumed that a force necessary for forming the staple is equal, the stapler A of this embodiment has a clearly smaller formation load than the staplers B, C of other companies. Then, the reason why the formation load of the stapler A of this embodiment is smaller can be said to be that the drive portion and guide portion which make sliding contact with the ram when the staple is formed are composed of synthetic resin.

### [B]

Although the load is reduced if the power point magnification is increased, the power point magnification is preferred to be less than 5 times in views of requested characteristics such as ease of gripping, ease of maintenance and transportation, a small quantity of waste and the like. Because the power point magnification is 4.18 times in this embodiment, the formation load is reduced while the requested characteristic is satisfied.

A pair of the legs 6b provided on the ram 6 form the staple 3 supported by the anvil 5 and opposing faces of the leg 6b across the anvil 5 are formed as a formation face 6c. Particularly, a gap C between the formation face 6c of the leg 6b and an end portion of the anvil 5 opposing the formation face 6c is formed in a dimension corresponding to the size of the staple 3.

The dimension corresponding to the size of the staple 3 does not mean an equal dimension to the size of the staple 3 but how large clearance is to be provided to the size of the staple 3. The value of the gap C is not related to a dimensional tolerance set to the width dimension of the anvil 4 and a dimensional tolerance set between the formation faces 6c of a pair of the legs 6b formed on the ram 6. That is, the gap C is set up positively to enable the staple 3 to be formed into a shape favorable for suturing the body tissue and obtain such a dimension which minimizes a force to be applied to the lever 2.

Thus, the gap between the formation face 6c of the leg and the end portion between the leg and the anvil 5 is set to a variety of values and the relationship between a formation shape of the staple 3 (a gap between the pointed ends when the staple is formed into a square) and a force applied to the lever 2 is obtained through experiments and the value of the gap C is determined from the relationship between the formation shape and the force.

The formation shape of the staple 3 is a square in which the bent portions of the crown 3b are formed accurately substantially at right angle with the pointed ends 3a separated opposing each other with a slight gap. Further, a distance from the pointed end 3a of the staple 3 to the crown 3b is substantially 1/2 the width dimension of the anvil 5.

The reason why the bent portions of the crown 3b are substantially at right angle is that the formed staple 3 is not formed accurately at right angle due to an influence of spring back which occurs at the same time when capture by the ram 6 is released. Further, the gap between the anvil 5 and the pointed end 3a may be equal to the amount of spring back generated when the ram 6 is moved up to its moving limit in the direction of the anvil 5 to form the staple 3 (when the formation is completed) with the pointed ends 3a making no contact with each other and then the ram 6 is retreated from the anvil 5 (when the capture by the ram 6 is released from the staple 3).

The reason why the pointed ends 3a are protected frommaking contact with each other when the formation is completed is that when the pointed end 3a of the staple makes contact with the body of a patient upon suturing of the body tissue, bacteria is estimated to adhere to this pointed end and that if the pointed ends 3a with adhering bacteria make contact with each other in the body tissue, bacteria can move through the pointed ends 3a, which is not preferable.

The reason why it is favorable in the formed staple that the bent portions of the crown 3b are accurately at right angle is that the staple 3 cannot be separated from an affected area easily after the body tissue is sutured. For example, if the bent portion of the crown 3b is curved (in roundness), it becomes easier to escape from the affected area as time elapses after the suturing operation is executed.

The gap by spring back when the ram 6 is retreated with the pointed ends 3a not in contact with each other at the time of completion of formation in which the crown 3b of the staple 3 is bent substantially at right angle is about 0.3 mm to 0.5 mm. When the gap between the pointed ends 3a is smaller than 0.3 mm, the pointed ends 3a make contact with each other when the formation is completed and if the gap between the pointed ends 3a is larger than 0.5 mm, the bent portions of the crown 3b turn to an obtuse angle larger than the right angle.

The inventor and other people produced test pieces by changing the value of the gap C between the leg 6b formed on the ram 6 and the end portion of the anvil 5. With a staple 3 supported by the anvil 5, the ram 6 was moved to the anvil 5 to form the staple 3 and then, a necessary force applied to the lever 2 at this time was measured. Additionally, the gap between the pointed ends 3a of the staple 3 after it was formed and left the anvil 5 was measured.

As a result of this experiment, when the gap C was in a range of 1.01 times to 1.07 times the size of the staple 3, the bent portion at the crown 3b of the staple turns to substantially right angle and the gap between the pointed ends 3a was about 0. 32 mm to 0.43 mm. Further, the necessary force for formation was not so large.

Next, upon forming the staple 3 0.58 mm in thick, a sample 1 in which the gap C was set in a range of 0.610 mm to 0.633 mm (about 1.05 - 1.09 times the size of the staple), a sample 2 in which the gap C was set in a range of 0.598 mm to 0.620 mm (about 1.03 times to 1.07 times the size of the staple), a sample 3 in which the gap C was set in a range of 0.585 mm to 0.608 mm (about 1.01 to 1.05 times the size of the staple), a sample 4 in which the gap C was set in a range of 0.573 mm to 0.595 mm (about 0.99 to 1.02 times the size of the staple) and a sample 5 in which the gap C was set in a range of 0.560 mm to 0.583 mm (about 0.97 to 1.005 times the size of the staple) were produced. A force needed for forming the staple 3 supported by the anvil 5, a gap between the pointed ends 3a of the formed staple 3 and shape of the staple 3 according to these samples 1-5 will be described. The quantity of the staples 3 formed for each sample is 20.

In case of the sample 1, the force (average value, which is same in the following) was 2.55 kgf, the gap between the pointed ends (average value, which is same in the following) was 0.52 mm and the bent portion of the crown 3b was bent irregularly. Thus, the formed shape had a small problem although the force necessary for the formation was small.

In case of the sample 2, the force was 2.65 kgf, the gap between the pointed ends was 0.46 mm and the bent portion of the crown 3b was bent at right angle to such an extent that is satisfactory. Thus, the formed shape is satisfactory as a shape for suturing the body tissue.

In case of the sample 3, the force was 2.86 kgf, the gap between the pointed ends was 0.32 mm and the bent portion of the crown 3b was bent at right angle to such an extent that is sufficiently satisfactory. Thus, the formed shape is satisfactory as a shape for suturing the body tissue.

In case of the sample 4, the force was 2.96 kg, the gap between the pointed ends was 0.23 mm and the bent portion of the crown 3b was bent at right angle to such an extent that is sufficiently satisfactory. Although in case of the sample 4, the formed shape is satisfactory as a shape for suturing the body tissue, the gap between the pointed ends 3a is so small that the pointed ends 3a can make contact with each other when the formation is completed, which is not preferable.

In case of the sample 5, the force was 3.20 kg, the gap between the pointed ends 3a was 0.12 mm and the bent portion of the crown 3b was bent at right angle to such an extent that was sufficiently satisfactory. Although in case of the sample 5, the formed shape is satisfactory as a shape for suturing the body tissue, the gap between the pointed ends 3a is so small that the pointed ends 3a can make contact with each other when the formation is completed, which is not preferable. Further, the sample 5 has such a problem that the force necessary for formation is large.

Considering a result of the above-described experiment, although the gap C between the leg 6b and the end portion of the anvil 5 changes corresponding to the size of the staple 3, it is preferred to be in a range of 1.01 to 1.07 times the size of the staple 3. Although a preferable formed shape can be obtained if the gap C is smaller than 1.01 times the size of the stapler 3, the force necessary for the formation is increased. Further, although the force necessary for the formation is decreased if the gap C is larger than 1.07 times the size of the stapler 3, it is difficult to obtain any preferable formed shape.

In the stapler A having the above-described structure, in an initial condition in which no force is applied to the lever 2, as shown in FIG. 3A, the staple 3 is supported by the anvil 5 and the leg 6b of the ram 6 applies no force to the staple 3.

When a medical doctor grips the lever 2 to apply a force thereto, the lever 2 is rotated counterclockwise so that the ram 6 moves downward accompanied by this rotation and as shown in FIG. 3B, a bending force is applied from the leg 6b to the staple 3. As a result, the crown 3b of the staple 3 is curved like a bow.

Accompanied by a further downward moving of the ram 6, the crown 3b of the staple 3 is nipped between the end portion of the anvil 5 and the formation face 6c of the leg 6b so that the staple 3 is bent at right angle whereby suturing the body tissue in this bending process. Because the gap C between the formation face 6c and the end portion of the anvil 5 is formed in such a dimension corresponding to the size of the staple 3 which forms the staple 3 into a favorable shape and minimizes the necessary force for obtaining this formed shape, the favorable formation can be carried out without inducing an excessive fatigue in a medical doctor.

When the application of force to the lever 2 is released after the body tissue of an affected area is sutured by forming the staple 3 supported by the anvil 5, the ram 6 is moved upward by the urging force of the urging member 7, so that capture of the staple 3 supported by the anvil 5 is released after the formation is completed. Accompanied by this release, after the formation is completed, the staple 3 is left at the affected area through the opening portion 1c of the housing 1 to maintain the sutured condition.

Suturing of the body tissue is completed by repeating the above-described operation.

The stapler of the present invention can achieve suturing operation securely with a small force when the incisedbody tissue is sutured. Thus, the present invention is useful for suturing the incised portion in surgical operation.

## Claims

1. A medical stapler for suturing body tissue by forming a medical staple supported by an anvil by bringing a ram close to the anvil, comprising:
a housing provided with a ram guide portion for guiding the ram in the direction of the anvil and equipped with a lever such that it is rotatable;
a lever in which a drive portion formed in part thereof makes contact with the ram so as to bring the ram close to the anvil accompanied by a rotation of the lever;
an anvil that is fixed to the housing and continuous to the front end of an accommodating portion for accommodating a plurality of medical staples so as to support a medical staple supplied from the accommodating portion; and
a ram that is constructed to be capable of sliding with respect to the housing and reciprocating with respect to the anvil and has a contact portion making contact with the drive portion formed in part of the lever and a pair of legs formed on an end portion for forming the medical staple in the anvil,
wherein the ram is composed of metal material and the drive portion formed on part of the lever and the ram guide portion provided in the housing are composed of synthetic resin and a gap between a leg formed on the ram and the anvil opposing the leg is set to a dimension allowing the medical staple to be formed into an excellent shape with a light load.

2. The medical stapler according to claim 1, wherein the gap between the leg of the ram and the anvil is 1.01 to 1.07 times the size of the medical staple.

3. The medical stapler according to claim 2, wherein the formed shape of the staple is a square in which the bent portion of a crown is formed substantially at right angle.

4. The medical stapler according to claim 1-3, wherein when a distance from a rotation shaft (fulcrum point) of a lever to a drive portion (action point) for driving the ram is 1, the magnification of a distance from the rotation shaft (fulcrum point) to a grip portion (power point) is 5 times or less.
